(19) Europäisches Patentamt
European Patent Office
Office européen des brevets

(11) Veröffentlichungsnummer: **0 090 183 B1**

(12) # EUROPÄISCHE PATENTSCHRIFT

(45) Veröffentlichungstag der Patentschrift: **19.02.86**

(21) Anmeldenummer: **83101896.5**

(22) Anmeldetag: **26.02.83**

(51) Int. Cl.⁴: **C 07 C 121/52,** C 09 K 19/12, C 09 K 19/30, C 09 K 19/32, C 09 K 19/34 // C07D309/08, C07C49/327, C07C25/18, C07C43/225, C07C69/035, C07C63/313, C07C69/753

(54) **Polyhalogenaromaten.**

(30) Priorität: **13.03.82 DE 3209178**

(43) Veröffentlichungstag der Anmeldung: **05.10.83 Patentblatt 83/40**

(45) Bekanntmachung des Hinweises auf die Patenterteilung: **19.02.86 Patentblatt 86/8**

(84) Benannte Vertragsstaaten: **CH DE FR GB LI**

(56) Entgegenhaltungen:
**EP - A - 0 051 738**
**DE - A - 1 518 528**
**DE - A - 3 139 130**

**Kelker and Hatz, Handbook of liquid crystals (1980), S. 57-59**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Erlenweg 17, D-6110 Dieburg (DE)**
Erfinder: **Römer, Michael, Dr., Im Grossen Garten 18, D-6054 Rodgau (DE)**
Erfinder: **Weber, Georg, Wilhelm-Leuschner-Strasse 38, D-6106 Erzhausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 090 183 B1

**Beschreibung**

Die Erfindung betrifft neue Polyhalogenaromaten der Formel I

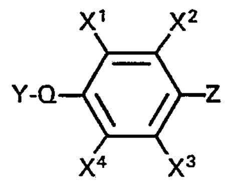

I

worin

Y und Z jeweils Alkyl, Alkoxy oder Alkanoyl mit jeweils 1 bis 12 C-Atomen, $-COOR^1$, $-O-CO-R^1$, CN, $NO_2$, $NH_2$ oder OH,

Q einen oder zwei gleiche oder verschiedene Reste ausgewählt aus der Gruppe bestehend aus Ph, Cy, 1,4-Bicyclo[2,2,2]octylen und 1,3-Dioxan-2,5-diyl,

$X^1$ bis $X^4$ jeweils H, F, Cl oder Br,

$R^1$ Alkyl mit 1 bis 4 C-Atomen, $Ph-R^2$ oder $Cy-R^2$,

$R^2$ Alkyl oder Alkoxy mit jeweils 1 bis 12 C-Atomen oder CN,

Ph 1,4-Phenylen und

Cy 1,4-Cyclohexylen

bedeuten

mit der Massgabe, dass höchstens einer der Reste $X^1$ und $X^2$ und höchstens einer der Reste $X^3$ und $X^4$ ein Wasserstoffatom bedeutet.

Diese Substanzen können wie ähnliche, z.B. aus der EP-OS 0 019 665 bekannte Verbindungen als Komponenten flüssig-kristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue flüssig-kristalline Verbindungen aufzufinden, die als Komponenten flüssig-kristalliner Dieletrika geeignet sind. Diese Aufgabe wurde durch die Bereitstellung der Verbindungen der Formel I gelöst.

Es wurde gefunden, dass die Verbindungen der Formel I vorzüglich als Komponenten flüssigkristalliner Dielektrika geeignet sind. Insbesondere gestatten sie grosse Variationsbreiten der dielektrischen Anisotropie und der optischen Anisotropie. Weiterhin zeigen sie hohe mittlere DK-Werte und damit ein hohes Lösungsvermögen für Leitsalze und für dichroitische Farbstoffe.

Die Verbindungen der Formel I sind in reinem Zustand farblos und bilden flüssigkristalline Mesophasen in einem für die elektrooptische Verwendung günstig gelegenen Temperaturbereich.

Diejenigen Verbindungen der Formel I, die optisch aktiv sind, eignen sich als chirale Dotierstoffe zur Herstellung cholesterischer Phasen wie sie für Farbstoff-Zellen nach White-Taylor verwendet werden können.

Gegenstand der Erfindung sind somit die Verbindungen der Formel I sowie ein Verfahren zu ihrer Herstellung, dadurch gekennzeichnet, dass man eine Verbindung der Formel II

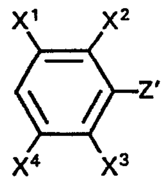

II

worin

Z' CN oder $NO_2$ bedeutet und

$X^1$ bis $X^4$ die angegebenen Bedeutungen haben

mit einem reaktionsfähigen Derivat einer Percarbonsäure der Formel III

$Y-Q-CO_3H$ III

worin

Y und Q die angegebenen Bedeutungen haben

umsetzt

und dass man gegebenenfalls in einer erhaltenen Verbindung der Formel I (Z = Z') den Rest Z' in eine Alkyl-, Alkoxy- oder Alkanoylgruppe mit jeweils 1 bis 12 C-Atomen, eine $NH_2$-, OH-, $COOR^1$- oder eine $-O-CO-R^1$-Gruppe überführt.

Weiterhin sind Gegenstand der Erfindung die Verwendung der Verbindungen der Formel I als Komponenten flüssigkristalliner Dielektrika, flüssigkristalline Dielektrika mit einem Gehalt an mindestens einer Verbindung der Formel I sowie elektrooptische Anzeigeelemente, die derartige Dielektrika enthalten.

Vor- und nachstehend haben Y, Z, Q, $X^1$ bis $X^4$, $R^1$, $R^2$, Ph, Cy und Z' die angegebene Bedeutung, sofern nicht ausdrücklich etwas anderes vermerkt ist.

Die Verbindungen der Formel I umfassen Verbindungen der folgenden Teilformeln Ia bis It:

| | |
|---|---|
| Y-Ph-W-Z | Ia |
| Y-Cy-W-Z | Ib |
| Y-Bi-W-Z | Ic |
| Y-Dio-W-Z | Id |
| Y-Ph-Ph-W-Z | Ie |
| Y-Ph-Cy-W-Z | If |
| Y-Ph-Bi-W-Z | Ig |
| Y-Ph-Dio-W-Z | Ih |
| Y-Cy-Ph-W-Z | Ii |
| Y-Cy-Cy-W-Z | Ij |
| Y-Cy-Bi-W-Z | Ik |
| Y-Cy-Dio-W-Z | Il |
| Y-Bi-Ph-W-Z | Im |
| Y-Bi-Cy-W-Z | In |
| Y-Bi-Bi-W-Z | Io |
| Y-Bi-Dio-W-Z | Ip |
| Y-Dio-Ph-W-Z | Iq |
| Y-Dio-Cy-W-Z | Ir |
| Y-Dio-Bi-W-Z | Is |
| Y-Dio-Dio-W-Z | It, |

worin der Kürze halber W für den $2-X^2-3-X^1-5-X^4-6-X^3$-1,4-phenylenrest, Bi für den 1,4-Bicyclo[2,2,2]octylenrest und Dio für den 1,3-Dioxan-2,5-diylrest steht.

Die Verbindungen der Formel Ib, ferner diejenigen der Formeln Ia, Ic, Ie, If, Ii und Ij sind bevorzugt.

In den Verbindungen der Formel I sowie Ib, Id, If, Ih bis Il, In und Ip bis It sind diejenigen Stereoisomeren bevorzugt, worin die beiden Substituenten an den Cyclohexylen- und den Dioxandiylresten jeweils in trans-Stellung zueinander stehen.

In den Verbindungen der Formel I sowie Ia bis It sind die Alkyl-, Alkoxy- und Alkanoylgruppen vorzugsweise geradkettig. Alkyl bedeutet vorzugsweise Ethyl, n-Propyl, n-Butyl, n-Pentyl oder n-Hexyl, weiterhin vorzugsweise Methyl, n-Heptyl, n-Octyl, n-Nonyl, n-Decyl, n-Undecyl oder n-Dodecyl. Alkoxy bedeutet vorzugsweise Methoxy, Ethoxy, n-Propoxy, n-Butoxy, n-Pentoxy oder n-Hexoxy, weiterhin bevorzugt n-Heptoxy, n-Octoxy, n-Nonoxy, n-Decyloxy, n-Undecyloxy oder n-Dodecyloxy. Alkanoyl bedeutet vorzugsweise Acetyl, Propionyl, Butyryl, Valeryl oder Hexanoyl, weiterhin bevorzugt Formyl, Heptanoyl, Octanoyl, Nonanoyl, Decanoyl, Undecanoyl oder Dodecanoyl.

Verbindungen der Formel I sowie Ia bis It mit verzweigten Alkyl-, Alkoxy- oder Alkanoylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl), Isobutyl (= 2-Methyl-propyl), 2-Methyl-butyl, Isopentyl (= 3-Methylbutyl), 2-Methylpentyl, 3-Methylpentyl, 3-Ethylhexyl, 2-Heptyl (= 1-Methylhexyl), 2-Octyl (= 1-Methylheptyl); bevorzugte verzweigte Alkoxyreste sind Isopropoxy, 2-Methyl-propoxy, 2-Methylbutoxy, 3-Methylbutoxy, 2-Methyl-pentoxy, 3-Methyl-pentoxy, 2-Ethyl-hexoxy, 1-Methyl-hexoxy, 1-Methyl-heptoxy; bevorzugte verzweigte Alkanoylreste sind Isobutyryl, 2-Methylbutyryl, 3-Methylbutyryl, 2-Methylpentanoyl, 3-Methylpentanoyl, 2-Ethylhexanoyl.

Im einzelnen bedeutet Y vorzugsweise Alkyl, Z vorzugsweise CN oder $NO_2$.

Der Rest W kann vorzugsweise 2,6-Di-Hal-, ferner 2,5-Di-Hal-, 3,5-Di-Hal-, 2,3,5-Tri-Hal-, 2,3,6-Tri-Hal- oder 2,3,5,6-Tetra-Hal-1,4-phenylen bedeuten, wobei Hal vorzugsweise F, weiterhin Cl oder Br bedeutet. Insbesondere ist W 2,6-Difluor-, 2-Chlor-6-fluor- oder 2,6-Dichlor-1,4-phenylen.

$R^1$ ist vorzugsweise geradkettiges Alkyl mit 1 bis 4 C-Atomen, Ph-$R^2$ oder Cy-$R^2$.

$R^2$ bedeutet vorzugsweise Alkyl mit insbesondere 3, 4 oder 5 C-Atomen; in der Gruppe Ph-$R^2$ bedeutet $R^2$ darüber hinaus bevorzugt Alkoxy mit insbesondere 1, 2, 3, 4 oder 5 C-Atomen oder CN.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

Man kann die Verbindungen der Formel I herstellen, indem man zunächst ein Benzonitril- (bevorzugt 2,6-Difluor-, 2-Chlor-6-fluor- oder 2,6-Dichlorbenzonitril) oder ein Nitrobenzolderivat (bevorzugt 2,6-Difluor-, 2-Chlor-6-fluor- oder 2,6-Dichlornitrobenzol) der Formel II mit einem reaktionsfähigen Derivat einer Percarbonsäure der Formel III (bevorzugt mit einem Ester der Formel Y-Q-$CO_3$Alkyl, der in situ aus dem entsprechenden Säurechlorid der Formel Y-Q-COCl und dem entsprechenden Alkylhydroperoxid gebildet werden kann und worin die Alkylgruppe insbesondere 1 bis 6 C-Atome enthält, bevorzugt aber tert.-Butyl bedeutet; oder mit einem Diacylperoxid der Formel Y-Q-CO-O-O-CO-Q-Y) umsetzt.

Diese Umsetzung wird zweckmässig in Gegenwart eines inerten Lösungsmittels vorgenommen, z.B. Benzol, im allgemeinen bei Temperaturen zwischen etwa 0 und etwa 150°, vorzugsweise zwischen 60 und 110°. Falls der Perester aus dem Säurechlorid in situ gebildet wird, ist zur Neutralisation des entstehenden Chlorwasserstoffs der Zusatz einer Base wie Pyridin oder Triethylamin empfehlenswert.

Die Ausgangsstoffe der Formeln II und III sowie ihre reaktionsfähigen Derivate sind zum grossen Teil bekannt, zum Teil können sie ohne Schwierigkeiten nach Standardverfahren der organischen Chemie aus literaturbekannten Verbindungen hergestellt werden.

Falls erwünscht, kann die CN- bzw. die $NO_2$-Gruppe in dem so erhaltenen Produkt der Formel I (Z = Z') in einen anderen Rest Z übergeführt werden.

So kann man beispielsweise ein erhaltenes Nitril (I, Z = CN) mit einer metallorganischen Verbindung der Formel R-M (worin R Alkyl mit 1 bis 11 C-Atomen und M MgCl, MgBr, MgJ oder Li bedeutet) zum entsprechenden Keton (I, Z = Alkanoyl mit 2 bis 12 C-Atomen) umsetzen, zweckmässig unter den üblichen Bedingungen einer Grignard-Reaktion oder einer Umsetzung mit Organo-Li-Verbindungen in einem inerten Lösungsmittel, z.B. einem Ether wie Diethylether, Diisopropylether oder Tetrahydrofuran oder einem Kohlenwasserstoff wie Benzol oder einem Gemisch derartiger Lösungsmittel bei Temperaturen zwischen etwa 0 und etwa 80°. Anschliessend hydrolysiert man, vorzugsweise in wässerigem saurem Medium, z.B. mit Salzsäure, Schwefelsäure oder $NH_4Cl$, bei Temperaturen zwischen etwa 0 und etwa 120°.

Aldehyde der Formel I (Z = CHO) sind z.B. aus den Nitrilen (I, Z = CN) mit $LiAlH(OC_2H_5)_3$ oder $NaAlH(OC_2H_5)_3$ in Diethylether oder Tetrahydrofuran oder mit $SnCl_2/HCl$ in Diethylether nach der Methode von Stephen erhältlich.

Verbindungen der Formel I, worin Z eine Alkylgruppe bedeutet, sind z.B. erhältlich durch Reduktion der Ketone (I, Z = Alkanoyl) nach den Methoden von Clemmensen (mit Zink, amalgamierten Zink oder Zinn und Salzsäure, zweckmässig in wässerig-alkanolischer Lösung oder in heterogener Phase mit Wasser/Benzol oder Wasser/Toluol bei Temperaturen zwischen etwa 80 und 120°) oder Wolff-Kishner (mit Hydrazin, zweckmässig in Gegenwart von Alkali wie KOH oder NaOH in einem hochsiedenden Lösungsmittel wie Diethylenglykol oder Triethylenglykol bei Temperaturen zwischen etwa 100 und 200°).

Amine (I, Z = $NH_2$) können durch Reduktion der Nitroverbindungen (I, Z = $NO_2$) hergestellt werden, beispielsweise durch Hydrierung an einem Metall-Katalysator wie Pt, Pd oder Raney-Ni, der auch in Form eines Oxids (z.B. $PtO_2$) oder auf einem Träger (z.B. Pd-Kohle, Pd/$CaCO_3$, Pd/$SrCO_3$) vorliegen kann, in einem inerten Lösungsmittel wie Methanol, Ethanol, Isopropanol, Ethylacetat, Tetrahydrofuran, Dioxan oder Essigsäure bei Temperaturen zwischen etwa 20 und 100° und Drücken zwischen etwa 1 und 200 bar, oder auch auf chemischem Wege, z.B. mit nascierendem Wasserstoff, der mit den Systemen Fe/HCl, Zn/NaOH, Zn/$CH_3COOH$ oder Sn/HCl erzeugt werden kann, mit $SnCl_2$/HCl, mit $H_2S$ oder Sulfiden oder mit $Na_2S_2O_4$.

Phenole (I, Z = OH) sind z.B. durch Diazotierung der Amine (I, Z = $NH_2$) und nachfolgende Verkochung erhältlich. Man kann wie üblich mit einem Salz oder einem Ester der salpetrigen Säure (wie $NaNO_2$ oder Butylnitrit) in saurem wässerigem Medium diazotieren und die erhaltene Diazoniumsalzlösung anschliessend durch Hydrolyse bei Temperaturen zwischen etwa 50 und 100° zersetzen.

Alkoxyverbindungen (I, Z = Alkoxy) sind durch Alkylierung der Phenole (I, Z = OH) erhältlich, wobei das Phenol zweckmässig zunächst in ein Phenolat, z.B. durch Behandeln mit NaOH, KOH, $Na_2CO_3$ oder $K_2CO_3$ in das entsprechende Alkalimetallphenolat übergeführt wird. Dieses kann dann mit dem entsprechendenn Alkylhalogenid, -sulfonat oder Dialkylsulfat umgesetzt werden, zweckmässig in einem inerten Lösungsmittel wie Aceton, Dimethylformamid oder Dimethylsulfoxid oder auch einem Überschuss an wässeriger oder wässerig-alkoholischer NaOH oder KOH bei Temperaturen zwischen etwa 20 und 100°.

Acylierung der Phenole (I, Z = OH) oder ihrer reaktionsfähigen Derivate mit Carbonsäuren der Formel $R^1$-COOH oder deren reaktionsfähigen Derivaten führt zu den entsprechenden Estern (I, Z = -O-CO-$R^1$). Ester der Formel I (Z = $COOR^1$) sind erhältlich durch Hydrolyse der Nitrile (I, Z = CN) zu den entsprechenden Carbonsäuren (I, Z = COOH), z.B. in alkalischem Medium mit wässerig-alkoholischem Alkali bei Temperaturen zwischen etwa 20 und etwa 100°, und nachfolgende Umsetzung dieser Carbonsäuren oder ihrer reaktionsfähigen Derivate mit Alkoholen oder Phenolen der Formel $R^1$OH oder mit deren reaktionsfähigen Derivaten.

Als reaktionsfähige Derivate der Carbonsäuren der Formeln I (Z = COOH) bzw. $R_1$-COOH eignen sich insbesondere die Säurehalogenide, vor allem die Chloride und Bromide, ferner die Anhydride sowie Azide. Als reaktionsfähige Derivate der Phenole der Formel I (Z = OH) bzw. der Alkohole der Formel $R^1$-OH kommen insbesondere die entsprechenden Metall-, z.B. Natrium- oder Kalium-phenolate bzw. -alkoholate in Betracht.

Die Veresterung wird vorteilhaft in Gegenwart eines inerten Lösungsmittels durchgeführt. Gut geeignet sind insbesondere Ether wie Diethylether, Di-n-butylether, Tetrahydrofuran, Dioxan oder Anisol, Ketone wie Aceton, Butanon oder Cyclohexanon, Amide wie Dimethylformamid oder Phosphorsäurehexamethyltriamid, Kohlenwasserstoffe wie Benzol Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuss einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden.

Die Reaktionstemperatur liegt gewöhnlich zwischen —50° und +250°, vorzugsweise zwischen —20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure der Formel II mit einem Alkohol oder Phenol der Formel III in der Regel in Gegenwart einer starken Säure, beispielsweise einer Mineralsäure wie Salzsäure oder Schwefelsäure, umgesetzt.

Eine bevorzugte Reaktionsweise ist die Umsetzung eines Säureanhydrids oder insbesondere eines Säurechlorids mit dem Phenol bzw. Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind.

Die erfindungsgemässen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexylcarbonsäurephenyl- oder -cyclohexylester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexyl-pyrimidine, Phenyl- oder Cyclohexyldioxane, gegebenenfalls halogenierten Stilbene, Benzylphenylether, Tolane und substituierten Zimtsäuren. Die wichtigsten als Bestandteile derartiger flüssigkristalliner Dielektrika in Frage kommenden Verbindungen lassen sich durch die Formel IV charakterisieren,

$$R^3\text{-A-G-E-}R^4 \qquad \text{IV}$$

worin A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CL- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-PH-COO- |

oder eine C-C-Einfachbindung,

L Halogen, vorzugsweise Chlor, oder CN, und $R^3$ und $R^4$ Alkyl, Alkoxy, Alkanoyloxy oder Alkoxycarbonyloxy mit bis zu 18, vorzugsweise bis zu 8 Kohlenstoffatomen, oder einer einer dieser Reste auch CN, NC, $NO_2$, $CF_3$, F, Cl oder Br bedeuten. Bei den meisten dieser Verbindungen sind $R^3$ und $R^4$ voneinander verschieden, wobei einer dieser Reste meist eine Alkyl- oder Alkoxygruppe ist. Viele solcher Substanzen oder auch Gemische davon sind im Handel erhältlich.

Die erfindungsgemässen Dielektrika enthalten etwa 0,1 bis 30, vorzugsweise 2 bis 25% einer oder mehrerer Verbindungen der Formel I.

Die Herstellung der erfindungsgemässen Dielektrika erfolgt in an sich üblicher Weise. In der Regel werden die Komponenten ineinander gelöst, zweckmässig bei erhöhter Temperatur. Wenn dabei eine Temperatur oberhalb des Klärpunkts des Hauptbestandteils gewählt wird, kann die Vollständigkeit des Lösungsvorgangs besonders leicht beobachtet werden.

Durch geeignete Zusätze können die flüssigkristallinen Dielektrika nach der Erfindung so modifiziert werden, dass sie in allen bisher bekannt gewordenen Arten von Flüssigkristallanzeigeelementen verwendet werden können.

Derartige Zusätze sind dem Fachmann geläufig und in der Literatur ausführlich beschrieben. Beispielsweise können dichroitische Farbstoffe oder Substanzen zur Veränderung der dielektrischen Anisotropie, der Viskosität, der Leitfähigkeit und/oder der Orientierung der nematischen Phasen zugesetzt werden. Derartige Substanzen sind z.B. in den DE-OS 2 209 127, 2 240 864, 2 321 632, 2 338 281, 2 450 088, 2 637 430, 2 853 728 und 2 902 177 beschrieben.

Die folgenden Beispiele sollen die Erfindung erläutern. In den Beispielen bedeuten F. den Schmelzpunkt und K. den Klärpunkt einer flüssigkristallinen Substanz. Vor- und nachstehend bedeuten Prozentangaben Gewichtsprozente; alle Temperaturen sind in Grad Celsius angegeben. «Übliche Aufarbeitung» bedeutet: Man gibt, falls erforderlich, Wasser und/oder ein organisches Lösungsmittel wie Toluol, $CH_2Cl_2$ oder $CHCl_3$ hinzu, trennt ab, dampft die organische Phase ein und reinigt den Rückstand durch Chromatographie (Kieselgel) und/oder Kristallisation.

*Beispiel 1*

Eine Lösung von 21,7 g trans-4-Pentylcyclohexancarbonsäurechlorid in 100 ml Benzol wird bei 10° mit 9 g tert.-Butylhydroperoxid und 7,9 g Pyridin versetzt. Nach einstündigem Rühren bei 10° filtriert man das gebildete Pyridin-hydrochlorid ab und gibt zu der Lösung des erhaltenen Peresters 13,9 g 2,6-Difluorbenzonitril hinzu. Nach 16stündigem Kochen, Abkühlen, Waschen mit Wasser und Eindampfen reinigt man chomatographisch (Kieselgel, Toluol) und erhält 2,6-Difluor-4-(trans-4-pentylcyclohexyl)-benzonitril, F. 15°, K. 0°.

Analog erhält man aus den entsprechenden Säurechloriden und den entsprechenden Benzonitrilen bzw. Nitrobenzolen:

2,5-Difluor-4-(trans-4-pentylcyclohexyl)-benzonitril
2,6-Difluor-4-(trans-4-methylcyclohexyl)-benzonitril
2,6-Difluor-4-(trans-4-ethylcyclohexyl)-benzonitril
2,6-Difluor-4-(trans-4-propylcyclohexyl)-benzonitril
2,6-Difluor-4-(trans-4-butylcyclohexyl)-benzonitril
2,6-Difluor-4-(trans-4-hexylcyclohexyl)-benzonitril
2,6-Difluor-4-(trans-4-heptylcyclohexyl)-benzonitril
2,6-Difluor-4-(trans-4-dodecylcyclohexyl)-benzonitril
3,5-Difluor-4-(trans-4-pentylcyclohexyl)-benzonitril
2-Chlor-6-fluor-4-(trans-4-methylcyclohexyl)-benzonitril
2-Chlor-6-fluor-4-(trans-4-methylcyclohexyl)-benzonitril
2-Chlor-6-fluor-4-(trans-4-ethylcyclohexyl)-benzonitril
2-Chlor-6-fluor-4-(trans-4-propylcyclohexyl)-benzonitril
2-Chlor-6-fluor-4-(trans-4-butylcyclohexyl)-benzonitril
2-Chlor-6-fluor-4-(trans-4-pentylcyclohexyl)-benzonitril
2-Chlor-6-fluor-4-(trans-4-hexylcyclohexyl)-benzonitril
2-Chlor-6-fluor-4-(trans-4-heptylcyclohexyl)-benzonitril
2,6-Dichlor-4-(trans-4-methylcyclohexyl)-benzonitril
2,6-Dichlor-4-(trans-4-ethylcyclohexyl)-benzonitril
2,6-Dichlor-4-(trans-4-propylcyclohexyl)-benzonitril
2,6-Dichlor-4-(trans-4-butylcyclohexyl)-benzonitril
2,6-Dichlor-4-(trans-4-pentylcyclohexyl)-benzonitril
2,6-Dichlor-4-(trans-4-hexylcyclohexyl)-benzonitril
2,6-Dichlor-4-(trans-4-heptylcyclohexyl)-benzonitril
2,6-Dibrom-4-(trans-4-pentylcyclohexyl)-benzonitril
2,3,5-Trifluor-4-(trans-4-pentylcyclohexyl)-benzonitril
2,3,6-Trifluor-4-(trans-4-pentylcyclohexyl)-benzonitril
2,3,5,6-Tetrafluor-4-(4-pentylcyclohexyl)-benzonitril
2,6-Difluor-4-[trans-4-(trans-4-metyhlcyclohexyl)-cyclohexyl]-benzonitril
2,6-Difluor-4-[trans-4-(trans-4-ethylcyclohexyl)-cyclohexyl]-benzonitril
2,6-Difluor-4-[trans-4-(trans-4-propylcyclohexyl)-cyclohexyl]-benzonitril
2,6-Difluor-4-[trans-4-(trans-4-butylcyclohexyl)-cyclohexyl]-benzonitril, F. 50°, K. 130°.

2,6-Difluor-4-[trans-4-(trans-4-pentylcyclohexyl)-cyclohexyl]-benzonitril
2,6-Difluor-4-[trans-4-(trans-4-hexylcyclohexyl)-cyclohexyl]-benzonitril
2,6-Difluor-4-[trans-4-(trans-4-heptylcyclohexyl)-cyclohexyl]-benzonitril
2-Chlor-6-fluor-4-[trans-4-(trans-4-methylcyclohexyl)-cyclohexyl]-benzonitril
2-Chlor-6-fluor-4-[trans-4-(trans-4-ethylcyclohexyl)-cyclohexyl]-benzonitril
2-Chlor-6-fluor-4-[trans-4-(trans-4-proylcyclohexyl)-cyclohexyl]-benzonitril
2-Chlor-6-fluor-4-[trans-4-(trans-4-butylcyclohexyl)-cyclohexyl]-benzonitril
2-Chlor-6-fluor-4-[trans-4-(trans-4-pentylcyclohexyl)-cyclohexyl]-benzonitril
2-Chlor-6-fluor-4-[trans-4-(trans-4-hexylcyclohexyl)-cyclohexyl]-benzonitril
2-Chlor-6-fluor-4-[trans-4-(trans-4-heptylcyclohexyl)-cyclohexyl]-benzonitril
2,6-Dichlor-4-[trans-4-(trans-4-methylcyclohexyl)-cyclohexyl]-benzonitril
2,6-Dichlor-4-[trans-4-(trans-4-ethylcyclohexyl)-cyclohexyl]-benzonitril
2,6-Dichlor-4-[trans-4-(trans-4-propylcyclohexyl)-cyclohexyl]-benzonitril
2,6-Dichlor-4-[trans-4-(trans-4-butylcyclohexyl)-cyclohexyl]-benzonitril
2,6-Dichlor-4-[trans-4-(trans-4-pentylcyclohexyl)-cyclohexyl]-benzonitril
2,6-Dichlor-4-[trans-4-(trans-4-hexylcyclohexyl)-cyclohexyl]-benzonitril
2,6-Dichlor-4-[trans-4-(trans-4-heptylcyclohexyl)-cyclohexyl]-benzonitril
2,6-Difluor-4-(trans-4-methyl-1,4-bicyclo[2,2,2]-octyl)-benzonitril
2,6-Difluor-4-(trans-4-ethyl-1,4-bicyclo[2,2,2]-octyl)-benzonitril
2,6-Difluor-4-(trans-4-propyl-1,4-bicyclo[2,2,2]-octyl)-benzonitril
2,6-Difluor-4-(trans-4-butyl-1,4-bicyclo[2,2,2]-octyl)-benzonitril
2,6-Difluor-4-(trans-4-pentyl-1,4-bicyclo[2,2,2]-octyl)-benzonitril
2,6-Difluor-4-(trans-4-hexyl-1,4-bicyclo[2,2,2]-octyl)-benzonitril
2,6-Difluor-4-(trans-4-heptyl-1,4-bicyclo[2,2,2]-octyl)-benzonitril
2-Chlor-6-fluor-4-(trans-4-methyl-1,4-bicyclo-[2,2,2]octyl)-benzonitril
2-Chlor-6-fluor-4-(trans-4-ethyl-1,4-bicyclo-[2,2,2]octyl)-benzonitril
2-Chlor-6-fluor-4-(trans-4-propyl-1,4-bicyclo-[2,2,2]octyl)-benzonitril
2-Chlor-4-fluor-4-(trans-4-butyl-1,4-bicyclo-[2,2,2]octyl)-benzonitril
2-Chlor-4-fluor-4-(trans-4-pentyl-1,4-bicyclo-[2,2,2]octyl)-benzonitril
2-Chlor-4-fluor-4-(trans-4-hexyl-1,4-bicyclo-[2,2,2]octyl)-benzonitril
2-Chlor-4-fluor-4-(trans-4-heptyl-1,4-bicyclo-[2,2,2]octyl)-benzonitril
2,6-Dichlor-4-(trans-4-methyl-1,4-bicyclo[2,2,2]-octyl)-benzonitril
2,6-Dichlor-4-(trans-4-ethyl-1,4-bicyclo[2,2,2]-octyl)-benzonitril
2,6-Dichlor-4-(trans-4-propyl-1,4-bicyclo[2,2,2]-octyl)-benzonitril
2,6-Dichlor-4-(trans-4-butyl-1,4-bicyclo[2,2,2]-octyl)-benzonitril
2,6-Dichlor-4-(trans-4-pentyl-1,4-bicyclo[2,2,2]-octyl)-benzonitril
2,6-Dichlor-4-(trans-4-hexyl-1,4-bicyclo[2,2,2]-octyl)-benzonitril
2,6-Dichlor-4-(trans-4-heptyl-1,4-bicyclo[2,2,2]-octyl)-benzonitril
2,6-Difluor-4-cyan-4′-methyl-biphenyl
2,6-Difluor-4-cyan-4′-ethyl-biphenyl
2,6-Difluor-4-cyan-4′-propyl-biphenyl
2,6-Difluor-4-cyan-4′-butyl-biphenyl
2,6-Difluor-4-cyan-4′-pentyl-biphenyl
2,6-Difluor-4-cyan-4′-hexyl-biphenyl
2,6-Difluor-4-cyan-4′-heptyl-biphenyl
2,6-Difluor-4-cyan-4′-methoxy-biphenyl
2,6-Difluor-4-cyan-4′-ethoxy-biphenyl
2,6-Difluor-4-cyan-4′-propoxy-biphenyl
2,6-Difluor-4-cyan-4′-butoxy-biphenyl
2,6-Difluor-4-cyan-4′-pentoxy-biphenyl
2,6-Difluor-4-cyan-4′-hexoxy-biphenyl
2,6-Difluor-4-cyan-4′-heptoxy-biphenyl
2-Chlor-4-cyan-6-fluor-4′-methyl-biphenyl
2-Chlor-4-cyan-6-fluor-4′-ethyl-biphenyl
2-Chlor-4-cyan-6-fluor-4′-propyl-biphenyl
2-Chlor-4-cyan-6-fluor-4′-butyl-biphenyl
2-Chlor-4-cyan-6-fluor-4′-pentyl-biphenyl
2-Chlor-4-cyan-6-fluor-4′-hexyl-biphenyl
2-Chlor-4-cyan-6-fluor-4′-heptyl-biphenyl
2-Chlor-4-cyan-6-fluor-4′-methoxy-biphenyl
2-Chlor-4-cyan-6-fluor-4′-ethoxy-biphenyl
2-Chlor-4-cyan-6-fluor-4′-propoxy-biphenyl
2-Chlor-4-cyan-6-fluor-4′-butoxy-biphenyl
2-Chlor-4-cyan-6-fluor-4′-pentoxy-biphenyl
2-Chlor-4-cyan-6-fluor-4′-hexoxy-biphenyl
2-Chlor-4-cyan-6-fluor-4′-heptoxy-biphenyl
2,6-Dichlor-4-cyan-4′-methyl-biphenyl
2,6-Dichlor-4-cyan-4′-ethyl-biphenyl
2,6-Dichlor-4-cyan-4′-propyl-biphenyl
2,6-Dichlor-4-cyan-4′-butyl-biphenyl
2,6-Dichlor-4-cyan-4′-pentyl-biphenyl
2,6-Dichlor-4-cyan-4′-hexyl-biphenyl
2,6-Dichlor-4-cyan-4′-heptyl-biphenyl
2,6-Dichlor-4-cyan-4′-methoxy-biphenyl
2,6-Dichlor-4-cyan-4′-ethoxy-biphenyl
2,6-Dichlor-4-cyan-4′-propoxy-biphenyl
2,6-Dichlor-4-cyan-4′-butoxy-biphenyl
2,6-Dichlor-4-cyan-4′-pentoxy-biphenyl
2,6-Dichlor-4-cyan-4′-hexoxy-biphenyl
2,6-Dichlor-4-cyan-4′-heptoxy-biphenyl
2,6-Difluor-4-cyan-4″-propyl-terphenyl
2,6-Difluor-4-cyan-4″-butyl-terphenyl
2,6-Difluor-4-cyan-4″-pentyl-terphenyl
2,6-Difluor-4-(trans-4-p-propylphenyl-cyclohexyl)-benzonitril
2,6-Difluor-4-(trans-4-p-butylphenyl-cyclohexyl)-benzonitril
2,6-Difluor-4-(trans-4-p-pentylphenyl-cyclohexyl)-benzonitril
2,6-Difluor-4-cyan-4′-(trans-4-propylcyclohexyl)-biphenyl

2,6-Difluor-4-cyan-4'-(trans-4-putylcyclohexyl)-biphenyl
2,6-Difluor-4-cyan-4'-(trans-4-pentylcyclohexyl)-biphenyl
2,6-Difluor-4-(trans-4-ppropylcyclohexyl)-nitrobenzol
2,6-Difluor-4-(trans-4-butylcyclohexyl)-nitrobenzol
2,6-Difluor-4-(trans-4-pentylcyclohexyl)-nitrobenzol
2,6-Difluor-4-[trans-4-(trans-4-propylcyclohexyl)-cyclohexyl]-nitrobenzol
2,6-Difluor-4-[trans-4-(trans-4-butylcyclohexyl)-cyclohexyl]-nitrobenzol
2,6-Difluor-4-[trans-4-(trans-4-pentylcyclohexyl)-cyclohexyl]-nitrobenzol
2,6-Difluor-4-nitro-4'-propyl-biphenyl
2,6-Difluor-4-nitro-4'-butyl-biphenyl
2,6-Difluor-4-nitro-4'-pentyl-biphenyl.

*Beispiel 2*

Analog Beispiel 1 erhält man mit 5-Butyl-1,3-dioxan-2-carbonsäurechlorid (erhältlich durch Reaktion von Glyoxylsäureethylester mit 2-Butylpropan-1,3-diol zu 5-Butyl-1,3-dioxan-2-carbonsäureethylester, Verseifung und Umsetzung der erhaltenen freien Säure mit $SOCl_2$) das 2-(3,5-Difluor-4-cyanphenyl)-5-butyl-1,3-dioxan.

Analog sind erhältlich:
2-(3,5-Difluor-4-cyanphenyl)-5-propyl-1,3-dioxan
2-(3,5-Difluor-4-cyanphenyl)-5-pentyl-1,3-dioxan
2-(3,5-Dichlor-4-cyanphenyl)-5-propyl-1,3-dioxan
2-(3,5-Dichlor-4-cyanphenyl)-5-butyl-1,3-dioxan
2-(3,5-Dichlor-4-cyanphenyl)-5-pentyl-1,3-dioxan.

*Beispiel 3*

Man bereitet aus 28,4 g $CH_3J$ und 4,86 g Mg in 200 ml Ether eine Grignard-Lösung, ersetzt den grössten Teil des Ethers durch 160 ml Benzol, gibt 29,1 g 2,6-Difluor-4-(trans-4-pentylcyclohexyl)-benzonitril hinzu und kocht 3 Stunden. Man kühlt auf 0°, gibt 120 ml kalte 6 n Salzsäure hinzu, kocht 8 Stunden, kühlt ab, arbeitet wie üblich auf und erhält 2,6-Difluor-4-(trans-4-pentylcyclohexyl)-acetophenon.

Analog erhält man durch Reaktion der entsprechenden Nitrile mit Alkylmagnesiumhalogeniden und nachfolgende Hydrolyse:
2,6-Difluor-4-(trans-4-propylcyclohexyl)-acetophenon
2,6-Difluor-4-(trans-4-butylcyclohexyl)-acetophenon
2,6-Difluor-4-(trans-4-propylcyclohexyl)-propiophenon
2,6-Difluor-4-(trans-4-butylcyclohexyl)-propiophenon
2,6-Difluor-4-(trans-4-pentylcyclohexyl)-propiophenon
2,6-Difluor-4-(trans-4-propylcyclohexyl)-butyrophenon
2,6-Difluor-4-(trans-4-butylcyclohexyl)-butyrophenon
2,6-Difluor-4-(trans-4-pentylcyclohexyl)-butyrophenon
2,6-Difluor-4-(trans-4-propylcyclohexyl)-valerophenon
2,6-Difluor-4-(trans-4-butylcyclohexyl)-valerophenon
2,6-Difluor-4-(trans-4-pentylcyclohexyl)-valerophenon.

*Beispiel 4*

Ein Gemisch aus 30,8 g 2,6-Difluor-4-(trans-4-pentylcyclohexyl)-acetophenon, 15 g KOH, 25 ml 85%igem Hydrazin und 250 ml Diethylenglykol wird 1 Stunde auf 100° erwärmt. Man steigert die Temperatur langsam bis zur Zersetzung des Hydrazons, kocht noch 4 Stunden, kühlt ab, arbeitet wie üblich auf und erhält 1-Ethyl-2,6-difluor-4-(trans-4-pentylcyclohexyl)-benzol.

Analog erhält man durch Wolff-Kishner-Reduktion der entsprechenden Ketone:
1-Ethyl-2,6-difluor-4-(trans-4-propylcyclohexyl)-benzol
1-Ethyl-2,6-difluor-4-(trans-4-butylcyclohexyl)-benzol
1-Propyl-2,6-difluor-4-(trans-4-propylcyclohexyl)-benzol
1-Propyl-2,6-difluor-4-(trans-4-butylcyclohexyl)-benzol
1-Propyl-2,6-difluor-4-(trans-4-pentylcyclohexyl)-benzol
1-Butyl-2,6-difluor-4-(trans-4-propylcyclohexyl)-benzol
1-Butyl-2,6-difluor-4-(trans-4-butylcyclohexyl)-benzol
1-Butyl-2,6-difluor-4-(trans-4-pentylcyclohexyl)-benzol
1-Pentyl-2,6-difluor-4-(trans-4-propylcyclohexyl)-benzol
1-Pentyl-2,6-difluor-4-(trans-4-butylcyclohexyl)-benzol
1-Pentyl-2,6-difluor-4-(trans-4-pentylcyclohexyl)-benzol.

*Beispiel 5*

a) Man löst 31,1 g 2,6-Difluor-4-(trans-4-pentylcyclohexyl)-nitrobenzol in 450 ml Isopropanol und hydriert an 0,5 g $PtO_2$ bei 20° und Normaldruck bis zur Aufnahme der berechneten Menge Wasserstoff. Es wird filtriert und eingedampft. Das erhaltene rohe 2,6-Difluor-4-(trans-4-pentylcyclohexyl)-anilin wird in einem Gemisch von 25 ml konzentrierter Schwefelsäure, 75 ml Dioxan und 75 ml Wasser gelöst und bei 3 bis 6° mit einer Lösung von 8 g $NaNO_2$ in 15 ml Wasser diazotiert. Das so erhaltene Gemisch wird portionsweise unter Rühren in 500 ml siedendes Wasser eingetragen. Man kocht noch 30 Minuten, kühlt ab, filtriert das erhaltene 2,6-Difluor-4-(trans-4-pentylcyclohexyl)-phenol ab und löst es in 250 ml 1 n Natronlauge. Portionsweise wird 12,6 g Dimethylsulfat zugegeben und anschliessend 1 Stunde auf 100° erhitzt. Nach dem Abkühlen und üblicher Aufarbeitung erhält man 2,6-Difluor-4-(trans-4-pentylcyclohexyl)-anisol.

b) Ein Gemisch von 28,2 g 2,6-Difluor-4-(trans-4-pentylcyclohexyl)-phenol, 6,9 g $K_2CO_3$, 25 g Hexyljodid und 250 ml Dimethylformamid wird unter Rühren 16 Stunden auf 80° erhitzt, dann abgekühlt und wie üblich aufgearbeitet. Man erhält 2,6-Difluor-4-(trans-4-pentylcyclohexyl)-1-hexoxybenzol.

Analog erhält man über
2,6-Difluor-4-(trans-4-propylcyclohexyl)-anilin,
2,6-Difluor-4-(trans-4-butylcyclohexyl)-anilin bzw.
2,6-Difluor-4-(trans-4-pentylcyclohexyl)-anilin

sowie

2,6-Difluor-4-(trans-4-propylcyclohexyl)-phenol,
2,6-Difluor-4-(trans-4-butylcyclohexyl)-phenol bzw.
2,6-Difluor-4-(trans-4-pentylcyclohexyl)-phenol

durch Veretherung:

2,6-Difluor-4-(trans-4-propylcyclohexyl)-1-methoxybenzol
2,6-Difluor-4-(trans-4-propylcyclohexyl)-1-ethoxybenzol
2,6-Difluor-4-(trans-4-propylcyclohexyl)-1-propoxybenzol
2,6-Difluor-4-(trans-4-propylcyclohexyl)-1-butoxybenzol
2,6-Difluor-4-(trans-4-propylcyclohexyl)-1-pentoxybenzol
2,6-Difluor-4-(trans-4-butylcyclohexyl)-1-methoxybenzol
2,6-Difluor-4-(trans-4-butylcyclohexyl)-1-ethoxybenzol
2,6-Difluor-4-(trans-4-butylcyclohexyl)-1-propoxybenzol
2,6-Difluor-4-(trans-4-butylcyclohexyl)-1-butoxybenzol
2,6-Difluor-4-(trans-4-butylcyclohexyl)-1-pentoxybenzol
2,6-Difluor-4-(trans-4-pentylcyclohexyl)-1-methoxybenzol
2,6-Difluor-4-(trans-4-pentylcyclohexyl)-1-ethoxybenzol
2,6-Difluor-4-(trans-4-pentylcyclohexyl)-1-propoxybenzol
2,6-Difluor-4-(trans-4-pentylcyclohexyl)-1-butoxybenzol
2,6-Difluor-4-(trans-4-pentylcyclohexyl)-1-pentoxybenzol.

*Beispiel 6*

Zu einer Lösung von 28,2 g 2,6-Difluor-4-(trans-4-pentylcyclohexyl)-phenol und 20 ml Pyridin in 300 ml Toluol gibt man 8 g Acetylchlorid und erwärmt 1 Stunde auf 80°. Nach Abkühlen und üblicher Aufarbeitung erhält man 2,6-Difluor-4-(trans-4-pentylcyclohexyl)-phenyl-acetat.

Analog erhält man durch Veresterung der entsprechenden Phenole:
2,6-Difluor-4-(trans-4-propylcyclohexyl)-phenyl-acetat
2,6-Difluor-4-(trans-4-butylcyclohexyl)-phenyl-acetat.

*Beispiel 7*

a) Man kocht eine Lösung von 10 g 2,6-Difluor-4-(trans-4-pentylcyclohexyl)-benzonitril und 20 g KOH in 40 ml Wasser und 160 ml Ethanol 16 Stunden, kühlt ab, arbeitet wie üblich auf und erhält 2,6-Difluor-4-(trans-4-pentylcyclohexyl)-benzoesäure.

b) Man lässt 10 g 2,6-Difluor-4-(trans-4-pentylcyclohexyl)-benzoesäure mit 200 ml methanolischer Salzsäure 24 Stunden bei 20° stehen, dampft ein und erhält 2,6-Difluor-4-(trans-4-pentylcyclohecyl)-benzoesäure-methylester.

c) Man kocht 31 g 2,6-Difluor-4-(trans-4-pentylcyclohexyl)-benzoesäure 1 Stunde mit 24 g $SOCl_2$, dampft ein, löst das erhaltene rohe Säurechlorid in 150 ml Toluol, versetzt mit 7,9 g Pyridin und 6 g Propanol und kocht 2 Stunden. Das Gemisch wird gekühlt und wie üblich aufgearbeitet. Man erhält 2,6-Difluor-4-(trans-4-pentylcyclohexyl)-benzoesäure-propylester.

Analog erhält man durch Veresterung:

2,6-Difluor-4-(trans-4-pentylcyclohexyl)-benzoesäure-(p-propylphenylester)
2,6-Difluor-4-(trans-4-pentylcyclohexyl)-benzoesäure-(p-methoxyphenylester)
2,6-Difluor-4-(trans-4-pentylcyclohexyl)-benzoesäure-(p-ethoxyphenylester)
2,6-Difluor-4-(trans-4-pentylcyclohexyl)-benzoesäure-(p-dodecyloxyphenylester)
2,6-Difluor-4-(trans-4-pentylcyclohexyl)-benzoesäure-(p-cyanphenylester)
2,6-Difluor-4-(trans-4-pentylcyclohexyl)-benzoesäure-(trans-4-propylcyclohexylester).

Es folgen Beispiele für erfindungsgemässe Dielektrika mit einem Gehalt an mindestens einer Verbindung der Formel I:

*Beispiel A*

Ein Gemisch aus

24% 2,6-Difluor-4-(trans-4-pentylcyclohexyl)-benzonitril
36% p-(trans-4-Pentylcyclohexyl)-benzonitril
25% p-(trans-4-Heptylcyclohexyl)-benzonitril
15% 4'-(trans-4-Pentylcyclohexyl)-biphenyl-4-carbonitril

zeigt F. —5°, K. 57°.

*Beispiel B*

Ein Gemisch aus

17% 2,6-Difluor-4-(trans-4-pentylcyclohexyl)-benzonitril
23% 2,6-Difluor-4-{4-pentyl-bicyclo[2,2,2]octyl-(1)}-benzonitril
16% trans-1-(p-Ethoxyphenyl)-4-propylcyclohexan
12% trans-1-(p-Butoxyphenyl)-4-propylcyclohexan
22% 4-Ethyl-4'-(trans-4-pentylcyclohexyl)-biphenyl
10% 4-(trans-4-Pentylcyclohexyl)-4'-(trans-4-propylcyclohexyl)-biphenyl

zeigt F. —10°, K. 78°.

*Beispiel C*

Ein Gemisch aus

26% 2,6-Difluor-4-{4-pentyl-bicyclo[2,2,2]octyl-(1)}-benzonitril
30% 4-Pentyl-4'-cyan-biphenyl
14% 4-Heptyl-4'-cyan-biphenyl
6% 4''-Pentyl-4-cyan-terphenyl

13% trans-4-Propylcyclohexancarbonsäure-p--ethoxyphenylester·
11% 2-p-Cyanphenyl-5-pentyl-1,3-dioxan
zeigt F. —12°, K. 58°.

**Patentansprüche**

1. Polyhalogenaromaten der Formel I

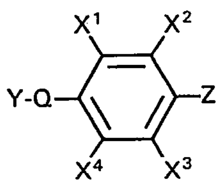

I

worin
Y und Z jeweils Alkyl, Alkoxy oder Alkanoyl mit jeweils 1 bis 12 C-Atomen, -COOR$^1$, -O-CO-R$^1$, CN, $NO_2$, $NH_2$ oder OH,
Q einen oder zwei gleiche oder verschiedene Reste ausgewählt aus der Gruppe bestehend aus Ph, Cy, 1,4-Bicyclo[2,2,2]octylen und 1,3-Dioxan-2,5--diyl,
X$^1$ bis X$^4$ jeweils H, F, Cl oder Br,
R$^1$ Alkyl mit 1 bis 4 C-Atomen, Ph-R$^2$ oder Cy-R$^2$,
R$^2$ Alkyl oder Alkoxy mit jeweils 1 bis 12 C-Atomen oder CN,
Ph 1,4-Phenylen und
Cy 1,4-Cyclohexylen
bedeuten
mit der Massgabe, dass höchstens einer der Reste X$^1$ und X$^2$ und höchstens einer der Reste X$^3$ und X$^4$ ein Wasserstoffatom bedeutet.

2. Verfahren zur Herstellung von Polyhalogenaromaten der Formel I nach Anspruch 1, dadurch gekennzeichnet, dass man eine Verbindung der Formel II

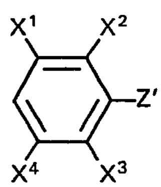

II

worin
Z' CN oder $NO_2$ bedeutet und
X$^1$ bis X$^4$ die angegebenen Bedeutungen haben
mit einem reaktionsfähigen Derivat einer Percarbonsäure der Formel III

$$Y\text{-}Q\text{-}CO_3H \qquad III$$

worin
Y und Q die angegebenen Bedeutungen haben
umsetzt
und dass man gegebenenfalls in einer erhaltenen Verbindung der Formel I (Z = Z') den Rest Z' in eine Alkyl-, Alkoxy- oder Alkanoylgruppe mit jeweils 1 bis 12 C-Atomen, eine $NH_2$-, OH-, COOR$^1$- oder -O-CO-R$^1$-Gruppe überführt.

3. Verwendung der Verbindungen der Formel I nach Anspruch 1 als Komponenten flüssigkristalliner Dielektrika für elektrooptische Anzeigeelemente.

4. Flüssigkristallines Dielektrikum für elektrooptische Anzeigeelemente mit mindestens zwei flüssigkristallinen Komponenten, dadurch gekennzeichnet, dass mindestens eine Komponente eine Verbindung der Formel I nach Anspruch 1 ist.

5. Elektrooptisches Anzeigeelement, dadurch gekennzeichnet, dass es ein Dielektrikum nach Anspruch 4 enthält.

**Claims**

1. Polyhalogenoaromatic compound of the formula I

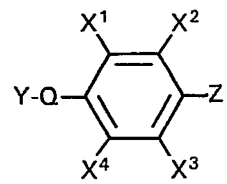

I

wherein Y and Z are each alkyl, alkoxy or alkanoyl having in each case 1 to 12 C atoms, -COOR$^1$, -O-CO-R$^1$, CN, $NO_2$, $NH_2$ or OH, Q is one or two identical or different radicals selected from the group comprising Ph, Cy, 1,4-bicyclo[2,2,2]octylene and 1,3-dioxane-2,5-diyl, X$^1$ to X$^4$ are each H, F, Cl or Br, R$^1$ is alkyl having 1 to 4 C atoms, Ph-R$^2$ or Cy-R$^2$, R$^2$ is alkyl or alkoxy having in each case 1 to 12 C atoms, or CN, Ph is 1,4-phenylene and Cy is 1,4-cyclohexylene, subject to the proviso that not more than one of the radicals X$^1$ and X$^2$ and not more than one of the radicals X$^3$ and X$^4$ is a hydrogen atom.

2. Process for the preparation of polyhalogenoaromatic compounds of the formula I according to Claim 1, characterised in that a compound of the formula II

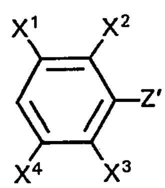

II

wherein Z' is CN or $NO_2$ and X$^1$ to X$^4$ have the meanings indicated, is reacted with a reactive derivative of a percarboxylic acid of the formula III

$$Y\text{-}Q\text{-}CO_3H \qquad III$$

wherein Y and Q have the meanings indicated, and, if desired, the radical Z' in a resulting compound of the formula I (Z = Z') is converted into an alkyl, alkoxy or alkanoyl group having in each case 1 to 12 C atoms, or into an $NH_2$, OH, COOR$^1$ or -O-CO-R$^1$ group.

3. The use of the compounds of the formula I according to Claim 1 as components of liquid-crystal dielectrics for eletrooptical display elements.

4. Liquid-crystal dielectric for electrooptical display elements having at least two liquid-cristalline components, characterised in that at least one component is a compound of the formula I according to Claim 1.

5. Electrooptical display element characterised in that it contains a dielectric according to Claim 4.

**Revendications**

1. Composés aromatiques polyhalogénés de formule I

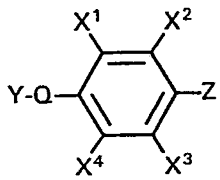

I

dans laquelle

Y et Z représentent chacun un groupe alkyle, alcoxy ou alcanoyle contenant chacun 1 à 12 atomes de carbone, $-COOR^1$, $-O-CO-R^1$, CN, $NO_2$, $NH_2$ ou OH,

Q représente un ou deux restes identiques ou différents choisis dans le groupe consistant en Ph, Cy, 1,4-bicyclo[2,2,2]octylène et 1,3-dioxanne-2,5-diyle,

$X^1$ à $X^4$ représentent chacun H, F, Cl ou Br,

$R^1$ représente un groupe alkyle en C1-C4, $Ph-R^2$ ou $Cy-R^2$,

$R^2$ représente un groupe alkyle en alcoxy contenant chacun un à douze atomes de carbone ou CN,

Ph représente le reste 1,4-phénylène et

Cy le reste 1,4-cyclohexylène,

étant spécifié qu'au maximum un des symboles $X^1$ et $X^2$ et au maximum un des symboles $X^3$ et $X^4$ représente un atome d'hydrogène.

2. Procédé de préparation de composés aromatiques polyhalogénés de formule I selon la revendication 1, caractérisé en ce que l'on fait réagir un composé de formule II

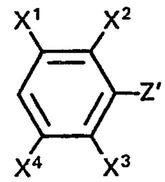

II

dans laquelle

Z' représente CN ou $NO_2$ et

$X^1$ à $X^4$ ont les significations indiquées ci-dessus, avec un dérivé réactif d'un acide percarboxylique de formule III

$$Y\text{-}Q\text{-}CO_3H \qquad III$$

dans laquelle Y et Q ont les significations indiquées ci-dessus, et dans un composé ainsi obtenu répondant à la formule I (Z = Z'), on convertit le cas échéant le reste Z' en un groupe alkyle, alcoxy ou alcanoyle contenant chacun 1 à 12 atomes de carbone, un groupe $N_2$, OH, $COOR^1$ ou $-O-CO-R^1$.

3. Utilisation des composés de formule I selon la revendication 1 en tant que composants de diélectriques à cristaux liquides pour éléments d'affichage électro-optiques.

4. Diélectrique à cristaux liquides pour éléments d'affichage électro-optiques à au moins deux composants à cristaux liquides, caractérisé en ce qu'un composant au moins consiste en un composé de formule I selon la revendication 1.

5. Élément d'affichage électro-optique caractérisé en ce qu'il contient un diélectrique selon la revendication 4.